# EUROPEAN PATENT APPLICATION

(11) **EP 4 428 150 A1**
(43) Date of publication of application: **11.09.2024**
(21) Application number: 23160599.9
(22) Date of filing: 07.03.2023
(51) Int. Cl.: C07K 16/18, C07K 16/26, A61K 39/00

(54) **ANTIBODY OR ANTIGEN-BINDING FRAGMENT THEREOF**

(71) Applicant: Peptide Logic LLC, San Diego, CA 92121 (US)
(72) Inventor: MACIUBA, Stephanie Elizabeth, San Diego, CA 92115 (US); BOWDEN, Gregory D, Chula Vista, CA 91913 (US); WISNIEWSKI, Kazimierz A., San Diego, CA 92126 (US); RIVIERE, Pierre Jean-Marie, San Diego, CA 92130 (US)
(74) Representative: Reddie & Grose LLP

(57) **Abstract**

An antibody or antigen-binding fragment thereof binds to human prolactin (hPRL) whilst also neutralizing its agonism of the human PRL receptor (hPRLR). Advantageously, this allows other hPRLR agonists to continue signalling. In turn, the antibody or antigen-binding fragment thereof is suitable for therapeutic use, e.g., as a composition comprising the antibody or antigen-binding fragment thereof. For example, the antibody or antigen-binding fragment thereof may be for use in the treatment of a disease or condition which is modulated by PRL, e.g., for use in the treatment of pain.

## Description

The invention relates to antibody or antigen-binding fragment thereof. More specifically, the invention relates to antibody or antigen-binding fragment thereof, that binds to human prolactin (hPRL).

### Background

Prolactin (PRL) is a widely expressed polypeptide hormone exerting pleiotropic endocrine, paracrine, and autocrine functions. PRL is produced by lactotroph cells of the anterior pituitary as well as by multiple extrapituitary tissues. PRL plays a critical role in mammogenesis and lactogenesis, and thus is naturally elevated during pregnancy and breastfeeding. Separately, excessive PRL production has been associated with galactorrhea, amenorrhea, mastalgia, infertility, endometriosis, osteoporosis, breast and prostate cancer, erectile dysfunction, alopecia, and migraine.

Circulating PRL levels are higher in women than men, increase during reproductive age and decline after menopause, and increase during pregnancy, suggesting control by female sex hormones. Additionally, PRL has emerged as a key contributor to sexual dimorphism in pain. PRL and PRL receptor (PRLR) are both expressed in trigeminal ganglia (TG) nociceptors of animals. PRLR is expressed at higher levels in TG nociceptors of female animals. PRL sensitizes female but not male TG nociceptors, causing release of calcitonin gene-related peptide (CGRP), a peptide known to trigger migraine attacks in humans. Topical application of PRL to the dura mater produces migraine-like pain in female but not male animals. Additionally, PRL also sensitizes dorsal root ganglia (DRG) nociceptors in female but not male animals. Locally applied and/or locally produced PRL produces hypersensitivity of nociceptors and pain in female but not male animals. Collectively, these data suggest that excessive PRL signaling may contribute to a broad range of female-predominant pain syndromes, and that blocking both pituitary and extrapituitary PRL may be clinically beneficial to treat pain in women.

Both production and secretion of PRL in pituitary and extrapituitary tissues are differentially regulated. In humans, there is a single gene coding for PRL and two distinct promoters differentially regulating PRL expression in pituitary and extrapituitary tissues. Furthermore, dopamine regulates PRL secretion in the pituitary but not in extrapituitary tissues.

Dopaminergic type 2 (D2) receptor agonists, such as cabergoline, used clinically to inhibit PRL release from the pituitary, have no utility to control PRL release from extrapituitary tissues.

Previous attempts at developing therapeutics able to block both pituitary and extrapituitary PRL responses have focused on PRLR antagonists, either peptides or anti-human PRLR (hPRLR) antibodies.

Peptide hPRLR antagonists, as disclosed in WO2018049092, have a very short half-life and thus could not be developed as therapeutics.

Antibodies binding the hPRLR, as disclosed in WO2012163932A1 or WO2008022295A2, prevent activation of hPRLR by hPRL. However, these hPRLR antibodies are not selective for hPRL, as they prevent ligand binding and/or activation of hPRLR signaling. Therefore, they are also likely to block hPRLR activation by growth hormone and placental lactogen, two other polypeptide hormones structurally related to hPRL, thus emphasizing the need for selective blockers of hPRL.

Antibodies targeting hPRL are commercially available as research tools. Whereas they generally bind hPRL, they are not always able to neutralize hPRL. Further, as these antibodies are all of mouse, rabbit, or goat origin, they are not suitable for therapeutic use.

Currently, there is no medication available that can solely and completely neutralize responses to hPRL produced by both pituitary and extrapituitary sites. As discussed above, current treatments and/or previous attempts at developing therapeutics to inhibit hPRL activity suffer various disadvantages that prevent them from blocking both pituitary and extrapituitary hPRL without inhibiting other hPRLR agonists.

The present invention describes novel, humanized anti-hPRL antibodies or antigen-binding fragments thereof, with demonstrated high affinity and selectivity for hPRL, and ability to potently and selectively inhibit hPRL activation of hPRLR.

### Summary of Invention

The invention relates to antibody or antigen-binding fragment thereof. More specifically, the invention relates to antibody or antigen-binding fragment thereof, wherein the antibody binds to hPRL.

Advantageously, the antibody or antigen-binding fragment thereof binds specifically to hPRL, allowing other hPRLR agonists to continue signalling.

According to a first aspect of the invention, there is provided an antibody or antigen-binding fragment thereof, wherein the antibody binds to hPRL. The antibodies of the present invention may be advantageous in the treatment of female pain. This is because female patients are at a greater risk of experiencing many clinical pain syndromes. Functional pain syndromes (FPS) make up a large subgroup of pain conditions defined by the absence of a clear etiology or tissue injury. FPS are characterized by unusually high female to male prevalence ratios. These include, but are not limited to, temporomandibular joint disorders, fibromyalgia, irritable bowel syndrome, chronic pelvic pain, interstitial cystitis, and migraine, and female-exclusive FPS such as dysmenorrhea, endometriosis, and vulvodynia. Female FPS typically peak during reproductive age, are often exacerbated during the menstrual cycle and by stress, and regress or disappear after menopause, suggesting a possible involvement of stress and/or female hormones in sex disparities of FPS.

hPRL may be described as set out on NCBI under ID 5617, herein incorporated by reference.

The antibody or antigen-binding fragment thereof may be an isolated antibody, or isolated antigen-binding fragment thereof.

An "antibody" may be an immunoglobulin molecule comprising 2 heavy chains (HCs) and 2 light chains (LCs) interconnected by disulfide bonds. The amino terminal portion of each LC and HC includes a variable region of about 100-120 amino acids primarily responsible for antigen recognition via the complementarity-determining regions (CDRs) contained therein. The CDRs are interspersed with regions that are more conserved, termed framework regions (FRs). Each light chain variable region (LCVR) and heavy chain variable region (HCVR) is composed of 3 CDRs and 4 FRs, arranged from amino-terminus to carboxy-terminus in the following order: FR1, CDR1, FR2, CDR2, FR3, CDR3, FR4. The 3 CDRs of the LC are referred to as "LCDR1, LCDR2, and LCDR3," and the 3 CDRs of the HC are referred to as "HCDR1, HCDR2, and HCDR3." The CDRs contain most of the residues which form specific interactions with the antigen. That is, the CDRs contain most of the residues that are in contact with the antigen's residues. The functional ability of the antibody to bind a particular antigen is, thus, largely influenced by the amino acid residues within the six CDRs. Assignment of amino acids to CDR domains within the LCVR and HCVR regions of the antibody of the present invention is based on the well-known Kabat numbering convention (Kabat, *et al.,* 1971).

The expression "selected from" may be understood to mean "to select a part of an amino acid sequence from a longer amino acid sequence described in a SEQ ID NO". For example, the expression "LCDR1 has an amino acid sequence selected from SEQ ID NO: 8" means LCDR1 may have an amino acid sequence which is identical to SEQ ID NO: 8 or may have an amino acid sequence which is a selected part of an amino acid sequence from the longer amino acid sequence described in SEQ ID NO: 8.

Optionally, the antibody or antigen-binding fragment thereof comprises a LC comprising one or more of LC CDRs. The LC CDRs may be LCDR1, LCDR2, and/or LCDR3. LCDR1 may have an amino acid sequence selected from SEQ ID NO: 8. LCDR2 may have an amino acid sequence selected from SEQ ID NO: 9. LCDR3 may have an amino acid sequence selected from SEQ ID NO: 10. Preferably, LCDR1 has an amino acid sequence selected from SEQ ID NO: 8, LCDR2 has an amino acid sequence selected from SEQ ID NO: 9, and/or LCDR3 has an amino acid sequence selected from SEQ ID NO: 10.

Optionally, the antibody or antigen-binding fragment thereof comprises a HC comprising one or more of HC CDRs. The HC CDRs may be HCDR1, HCDR2, and HCDR3. HCDR1 may have an amino acid sequence selected from SEQ ID NO: 5. HCDR2 may have an amino acid sequence selected from SEQ ID NO: 6. HCDR3 may have an amino acid sequence selected from SEQ ID NO: 7. Preferably, HCDR1 has an amino acid sequence selected from SEQ ID NO: 5, HCDR2 has an amino acid sequence selected from SEQ ID NO: 6, and/or HCDR3 has an amino acid sequence selected from SEQ ID NO: 7.

Optionally, the CDRs of the antibody of the present invention are defined according to **Table 1,** which lists the CDR numbering conventions used to define the CDRs of the antibody of the present invention.

**Table 1.**

| **CDR** | **Starting Amino Acid Residue Defined By:** | **Ending Amino Acid Residue Defined By:** |
|---|---|---|
| **HCDR1** | Chothia | Kabat |
| **HCDR2** | Kabat | Kabat |
| **HCDR3** | Kabat/Chothia | Kabat/Chothia |
| **LCDR1** | Kabat | Kabat |
| **LCDR2** | Kabat/Chothia | Kabat |
| **LCDR3** | Kabat | Kabat |

Optionally, the antibody or antigen-binding fragment thereof comprises a LCVR. The LCVR may have an amino acid sequence selected from SEQ ID NO: 4. Optionally, the antibody or antigen-binding fragment thereof comprises a HCVR. The HCVR may have an amino acid sequence selected from SEQ ID NO: 3.

Optionally, the antibody or antigen-binding fragment thereof binds an epitope having an amino acid sequence selected from SEQ ID NO: 11.

The term "epitope" as used herein refers to the surface of an antigen that is in contact with the variable region of an antibody. In particular, the tertiary structure of hPRL is made up of bundled alpha helices connected by unstructured loops. The hPRL epitope described is a confirmational epitope of nonconsecutive residues, meaning that the antibody thereof interacts with a particular region of the hPRL tertiary structure. Therefore, the hPRL epitope is comprised of residues of separate alpha helices that are in close proximity in three-dimensional space.

Optionally, the antibody or antigen-binding fragment thereof is an antibody or antigen-binding fragment thereof of Immunoglobulin G (IgG), for example an IgG of subclass IgG1, IgG2, IgG3, and/or IgG4.

Optionally, the antibody or antigen-binding fragment comprises a known Fc region mutation. For example, the antibody or antigen-binding fragment may be IgG4(S228P). Optionally, the antibody or antigen-binding fragment may comprise decreased Fc effector functions. For example, the antibody or antigen-binding fragment may be IgG1(L235E), IgG1(L234A/L235A), IgG4(S228P/L235E), IgG1(L234A/L235A/P329G), IgG1(P331S/L234E/L235F), IgG1(D265A), IgG1(G237A), IgG1(E318A), IgG1(E233P), IgG1(G236R/L328R), IgG1(A330L), IgG1(D270A), IgG1(K322A), IgG1(P329A), IgG1 (P331A), IgG1 (V264A), IgG1(F241A), IgG1 (N297A/G/Q), IgG4(S228P/F234A/L235A); and those reported to increase half-life, such as, IgG1(R435H), IgG1(N434A), IgG1(M252Y/S254T/T256E), IgG1(M428L/N434S), IgG1(T252L/T253S/T254F), IgG1(ΔE294/T307P/N434Y), IgG1(T256N/A378V/ S383N/N343Y), IgG1(ΔE294).

Optionally, the antibody or antigen-binding fragment thereof binds to PRL with an affinity of approximately 0.6 nM. Herein, "approximately" is understood to mean ±10%, preferably ±5%, preferably ±1%, preferably ±0.1%.

Preferably, the antibody or antigen-binding fragment thereof binds to hPRL with an affinity of 0.621 nM.

Optionally, the antibody or antigen-binding fragment thereof binds to hPRL with an affinity from 0.2 nM to 1.1 nM. Preferably, the antibody or antigen binding fragment thereof binds to hPRL with an affinity from 0.3 to 1 nM. More preferably, the antibody or antigen binding fragment thereof binds to hPRL with an affinity from 0.4 nM to 0.9 nM. More preferably, the antibody or antigen binding fragment thereof binds to hPRL with an affinity from 0.5 to 0.8 nM. More preferably, the antibody or antigen binding fragment thereof binds to hPRL with an affinity from 0.6 to 0.7 nM.

Optionally, the antibody or antigen binding fragment thereof binds to hPRL whereby the affinity is at least 1 nM. More preferably, the antibody or antigen binding fragment thereof binds to hPRL whereby the affinity is less than 1 nM. More preferably, the antibody or antigen binding fragment thereof binds to hPRL whereby the affinity is less than 0.7 nM.

The antibody or antigen-binding fragment thereof may bind to hPRL at an affinity comprising any range from the given endpoints.

Optionally, the antibody or antigen-binding fragment thereof binds to hPRL at an association rate kₒₙ of 3.47 × 10⁵ M⁻¹s⁻¹ and/or a dissociation rate k_{off} of 2.15 × 10⁻⁴s⁻¹.

Optionally, the antibody or antigen-binding fragment thereof binds to hPRL at an association rate kₒₙ of 1 × 10⁵ M⁻¹s⁻¹ to 6 × 10⁵ M⁻¹s⁻¹ and/or a dissociation rate k_{off} of 1 × 10⁻⁴s⁻¹ to 4 × 10⁻⁴s⁻¹. Preferably, the antibody or antigen-binding fragment thereof binds to hPRL at an association rate kₒₙ of 2 × 10⁵ M⁻¹s⁻¹ to 5 × 10⁵ M⁻¹s⁻¹ and/or a dissociation rate k_{off} of 2.5 × 10⁻⁴s⁻¹ to 3 × 10⁻⁴s⁻¹. Preferably, the antibody or antigen-binding fragment thereof binds to hPRL at an association rate kₒₙ of 3 × 10⁵ M⁻¹s⁻¹ to 4 × 10⁵ M⁻¹s⁻¹ and/or a dissociation rate k_{off} of 2 × 10⁻⁴s⁻¹ to 2.5 × 10⁻⁴s⁻¹.

Preferably, the antibody or antigen-binding fragment thereof binds to hPRL at an association rate kₒₙ of 3.1 × 10⁵ M⁻¹s⁻¹ to 3.9 × 10⁵ M⁻¹s⁻¹ and/or a dissociation rate k_{off} 1.7 × 10⁻⁴s⁻¹ to 2.5 × 10⁻⁴s⁻¹. More preferably, the antibody or antigen-binding fragment thereof binds to hPRL at an association rate kₒₙ of 3.2 × 10⁵ M⁻¹s⁻¹ to 3.8 × 10⁵ M⁻¹s⁻¹ and/or a dissociation rate k_{off} 1.8 × 10⁻⁴s⁻¹ to 2.4 × 10⁻⁴s⁻¹. More preferably, the antibody or antigen-binding fragment thereof binds to hPRL at an association rate kₒₙ of 3.3 × 10⁵ M⁻¹s⁻¹ to 3.7 × 10⁵ M⁻¹s⁻¹ and/or a dissociation rate k_{off} 1.0 × 10⁻⁴s⁻¹ to 2.3 × 10⁻⁴s⁻¹. More preferably, the antibody or antigen-binding fragment thereof binds to hPRL at an association rate kₒₙ of 3.4 × 10⁵ M⁻¹s⁻¹ to 3.6 × 10⁵ M⁻¹s⁻¹ and/or a dissociation rate k_{off} 2.0 × 10⁻⁴s⁻¹ to 2.2 × 10⁻⁴s⁻¹.

The antibody or antigen-binding fragment thereof may bind to hPRL at an association rate kₒₙ comprising any range from the given endpoints. The antibody or antigen-binding fragment thereof may bind to hPRL a dissociation rate k_{off} comprising any range from the given endpoints.

Optionally, the antibody or antigen-binding fragment thereof binds to PRL at an association rate kₒₙ of approximately 3.47 × 10⁵ M⁻¹s⁻¹ and/or a dissociation rate k_{off} of approximately 2.15 × 10⁻⁴s⁻¹. Herein, "approximately" is understood to mean ±10%, preferably ±5%, preferably ±1%, preferably ±0.1%.

Optionally, the antibody or antigen-binding fragment thereof neutralizes PRL-induced activation of PRLR and its downstream signaling pathway. The term "neutralize" as used herein refers to inhibition of PRLR activation by one of its natural agonists, specifically PRL. In particular, PRL activates PRLR in a concentration-dependent manner that eventually reaches a maximum plateau. PRLR activation herein is tracked by an increase in cytosolic phosphorylated STAT5, indicative of downstream signaling. Neutralizing efficacy of an antibody *in vitro* was assessed by challenging a submaximal activating concentration of PRL with a molar excess of antibody. Results were then compared to PRL alone controls to determine the percentage of neutralization. Lack of neutralization is evident by continued PRLR activation and a consistent response to agonist, which is independent of antibody concentration. Neutralizing antibodies were defined as those decreasing PRLR activation by a minimum of 75% of PRL alone. Preferably, addition of the antibody or antigen-binding fragment thereof to hPRL completely neutralizes hPRLR activation (95-100% decrease) such that treatment conditions are indistinguishable from those that did not receive the agonist. The strength of neutralization was assessed through a concentration-response curve of antibody challenged with a constant, submaximal concentration of PRL. The IC₅₀ values of antibodies were determined using a variable slope model of inhibition.

Optionally, the antibody or antigen-binding fragment thereof binds to hPRL, for example, with high affinity. The term "binds" as used herein refers to the protein-protein interaction of the antibody or antigen-binding fragment thereof with its antigen, hPRL. "Protein-protein interaction" refers to physical contact of two proteins facilitated by hydrophobic, electrostatic, and/or hydrogen interaction of amino acid side chains from two or more separate proteins. More specifically, binding of the antibody or antigen-binding fragment thereof is largely mediated and specified by a subset of the amino acids within the LCDRs and/or HCDRs. Further, the site of interaction on the antigen, hPRL, is defined as an epitope, as described above. Herein binding is assessed by the strength of interaction, or affinity, determined by biolayer interferometry, surface plasmon resonance, or similar techniques. The strength of interaction is expressed by the ratio of two kinetic rates. In particular, the quotient of the rate of dissociation (k_{off}) of the antigen from the antibody to the rate of association (kₒₙ) for the antibody with the antigen (K_{D}=k_{off}/kₒₙ), where a slower rate of dissociation is generally most indicative of increased affinity. Herein "high affinity" refers to 1 × 10⁻⁹ M and below. Binding "specificity" or "selectivity" as used herein refers to the ability of the antibody to discriminate between hPRL and other reference antigens, such as PRL from other species and other hormone agonists of hPRLR. Herein specificity is determined using biolayer interferometry and/or neutralization of agonist-induced activation of the hPRLR signaling pathway, as described above.

Optionally, the antibody or antigen-binding fragment thereof neutralizes hPRL at an *in vitro* efficacy of lower than IC₅₀ of 3.1 nM.

Preferably, the antibody or antigen-binding fragment thereof neutralizes hPRL at an *in vitro* efficacy of lower than IC₅₀ of 10 nM, preferably lower than IC₅₀ of 5 nM, preferably lower than IC₅₀ of 4 nM, preferably lower than IC₅₀ of 3.5 nM, preferably lower than IC₅₀ of 3.4 nM, preferably lower than IC₅₀ of 3.3 nM, preferably lower than IC₅₀ of 3.2 nM.

Optionally, the antibody or antigen-binding fragment thereof neutralizes hPRL at an *in vitro* efficacy of approximately IC₅₀ of 3.1 nM. Herein, "approximately" is understood to mean ±10%, preferably ±5%, preferably ±1%, preferably ±0.1%.

Advantageously, the antibody or antigen-binding fragment of the invention may provide an improved medication which solely neutralizes hPRL function. As set out above, known D2 receptor agonists, such as cabergoline, effectively inhibit pituitary secretion of hPRL but do not impact hPRL derived from extrapituitary tissues. hPRLR mAbs advantageously block all sources of hPRL from activating hPRLR, but their antagonism would also inhibit other hPRLR hormone agonists. The antibody or antigen-binding fragment thereof of the present invention selectively neutralizes hPRL activation of hPRLR, while allowing continued hPRLR activation by human growth hormone and human placental lactogen. Advantageously, this provides increased safety.

Optionally, LCDR1, LCDR2, LCDR3, HCDR1, HCDR2, and/or HCDR3 contain amino acid substitutions, deletions or additions. Preferably, the amino acid substitutions, deletions or additions are conservative sequence modifications. The term "conservative sequence modifications" refers to amino acid modifications that do not significantly affect or alter the binding characteristics of the antibody containing the amino acid sequence. Such conservative modifications include amino acid substitutions, additions and deletions. Modifications can be introduced into an antibody of the invention by standard techniques known in the art, such as site-directed mutagenesis and PCR-mediated mutagenesis. Conservative amino acid substitutions are ones in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine, tryptophan), nonpolar side chains (e.g., alanine, valine, leucine, isoleucine, proline, phenylalanine, methionine), beta-branched side chains (e.g., threonine, valine, isoleucine) and aromatic side chains (e.g., tyrosine, phenylalanine, tryptophan, histidine). Thus, one or more amino acid residues within the CDR regions of an antibody of the invention can be replaced with other amino acid residues from the same side chain family and the altered antibody can be tested for retained function (i.e., the functions set forth above) using the functional assays described herein.

Optionally, LCDR1 has an amino acid sequence selected from SEQ ID NO: 8 or an amino acid sequence having one, two, three, four or five amino acid substitutions, deletions or additions as compared to SEQ ID NO: 8.

Optionally, LCDR2 has an amino acid sequence selected from SEQ ID NO: 9 or an amino acid sequence having one, two, three, four or five amino acid substitutions, deletions or additions as compared to SEQ ID NO: 9.

Optionally, LCDR3 has an amino acid sequence selected from SEQ ID NO: 10 or an amino acid sequence having one, two, three, four or five amino acid substitutions, deletions or additions as compared to SEQ ID NO: 10.

Optionally, HCDR1 has an amino acid sequence selected from SEQ ID NO: 5 or an amino acid sequence having one, two, three, four or five amino acid substitutions, deletions or additions as compared to SEQ ID NO: 5.

Optionally, HCDR2 has an amino acid sequence selected from SEQ ID NO: 6 or an amino acid sequence having one, two, three, four or five amino acid substitutions, deletions or additions as compared to SEQ ID NO: 6.

Optionally, HCDR3 has an amino acid sequence selected from SEQ ID NO: 7 or an amino acid sequence having one, two, three, four or five amino acid substitutions, deletions or additions as compared to SEQ ID NO: 7.

Optionally, LCDR1 comprises amino acid sequence SEQ ID NO: 8 or an amino acid sequence that is at least 60% identical to SEQ ID NO: 8, preferably at least 65% identical, more preferably at least 70% identical, more preferably at least 75% identical, more preferably at least 80% identical, more preferably at least 85% identical, more preferably at least 90% identical, more preferably at least 95% identical, more preferably at least 98% identical, more preferably at least 99% identical to SEQ ID NO: 8.

Optionally, LCDR2 comprises amino acid sequence SEQ ID NO: 9 or an amino acid sequence that is at least 60% identical to SEQ ID NO: 9, preferably at least 65% identical, more preferably at least 70% identical, more preferably at least 75% identical, more preferably at least 80% identical, more preferably at least 85% identical, more preferably at least 90% identical, more preferably at least 95% identical, more preferably at least 98% identical, more preferably at least 99% identical to SEQ ID NO: 9.

Optionally, LCDR3 comprises amino acid sequence SEQ ID NO: 10 or an amino acid sequence that is at least 60% identical to SEQ ID NO: 10, preferably at least 65% identical, more preferably at least 70% identical, more preferably at least 75% identical, more preferably at least 80% identical, more preferably at least 85% identical, more preferably at least 90% identical, more preferably at least 95% identical, more preferably at least 98% identical, more preferably at least 99% identical to SEQ ID NO: 10.

Optionally, HCDR1 comprises amino acid sequence SEQ ID NO: 5 or an amino acid sequence that is at least 60% identical to SEQ ID NO: 5, preferably at least 65% identical, more preferably at least 70% identical, more preferably at least 75% identical, more preferably at least 80% identical, more preferably at least 85% identical, more preferably at least 90% identical, more preferably at least 95% identical, more preferably at least 98% identical, more preferably at least 99% identical to SEQ ID NO: 5.

Optionally, HCDR2 comprises amino acid sequence SEQ ID NO: 6 or an amino acid sequence that is at least 60% identical to SEQ ID NO: 6, preferably at least 65% identical, more preferably at least 70% identical, more preferably at least 75% identical, more preferably at least 80% identical, more preferably at least 85% identical, more preferably at least 90% identical, more preferably at least 95% identical, more preferably at least 98% identical, more preferably at least 99% identical to SEQ ID NO: 6.

Optionally, HCDR3 comprises amino acid sequence SEQ ID NO: 7 or an amino acid sequence that is at least 60% identical to SEQ ID NO: 7, preferably at least 65% identical, more preferably at least 70% identical, more preferably at least 75% identical, more preferably at least 80% identical, more preferably at least 85% identical, more preferably at least 90% identical, more preferably at least 95% identical, more preferably at least 98% identical, more preferably at least 99% identical to SEQ ID NO: 7.

In another aspect of the invention, there is provided a composition comprising the antibody or antigen-binding fragment thereof described herein, for example, a pharmaceutical composition comprising the antibody or antigen-binding fragment thereof described herein.

Optionally, the pharmaceutical composition comprises a pharmaceutically acceptable carrier, excipient, and/or diluent.

In another aspect of the invention, there is provided a composition (e.g., a pharmaceutical composition) comprising an antibody or antigen-binding fragment described herein, for use in the treatment of a disease or condition which is modulated by hPRL. Optionally, the composition comprising an antibody or antigen-binding fragment described herein is for use in the treatment of pain. Preferably, the composition comprising an antibody or antigen-binding fragment described herein is for use in the treatment of acute pain, neuropathic peripheral pain, chronic pain, and/or osteoarthritis.

The composition comprising an antibody or antigen-binding fragment described herein, for use in the treatment, may be administered to a subject or a patient. The patient may be a female patient. The patient may be a male patient. A pharmaceutical composition comprising an effective amount of the antibody or antigen-binding fragment may for administration to a patient, or administered to a patient, for example, a patient at risk for, or exhibiting, diseases or disorders as described herein by parental routes (e.g., subcutaneous, intravenous, intraperitoneal, intramuscular, or transdermal). An "effective amount" refers to an amount necessary (at dosages and for periods of time and for the means of administration) to achieve the desired therapeutic result. An effective amount of the antibody may vary according to factors such as the disease state, age, sex, and weight of the individual, and the ability of the antibody to elicit a desired response in the individual. An effective amount is also one in which any toxic or detrimental effects of the antibody of the present invention are outweighed by the therapeutically beneficial effects.

The antibody of the present invention can be used in the treatment of patients. More particularly the antibody of the present invention is expected to treat or prevent diseases or conditions which can be influenced by modulation of hPRL, for example for the treatment of pain, e.g., of acute pain, neuropathic peripheral pain, chronic pain, or osteoarthritis.

As used interchangeably herein, "treatment" and/or "treating" and/or "treat" are intended to refer to all processes wherein there may be a slowing, interrupting, arresting, controlling, stopping, or reversing of the progression of the disorders described herein, but does not necessarily indicate a total elimination of all disorder symptoms. Treatment includes administration of an antibody of the present invention for treatment of a disease or condition in a human that would benefit from a reduction in hPRL activity and includes: (a) inhibiting further progression of the disease; and (b) relieving the disease, i.e., causing regression of the disease or disorder or alleviating symptoms or complications thereof.

In line with normal meaning in the field, the expression "activity" is understood to mean the measure of biological function. In particular, "activity" as used herein refers to the extent of biological function of hPRL, hPRLR, and antibodies or antigen-binding fragments. Biological functions of hPRLR include but are not limited to cell surface receptor signaling, activation of JAK2 kinase activity, JAK-STAT cascade activity, binding PRL, binding growth hormone, and binding placental lactogen. Biological functions of hPRL include but are not limited to binding to PRLR and initiating its cell surface receptor signaling and downstream pathways. The biological function of antibodies or antigen-binding fragments thereof include but are not limited to binding to antigens, as described above. Further, as used herein, the impact of antigen binding by an antibody or antigen-binding fragment thereof can neutralize or inhibit the normal activity of the antigen, as described above.

In line with normal meaning in the field, the expression "efficacy" is understood to mean a measure of the extent of an antibody or antigen-binding fragment thereof to inhibit hPRL-induced activation of hPRLR. In particular, the maximal hPRLR activity may be obtained through addition of hPRL alone. Complete inhibition or neutralization of hPRL may be observed when addition of antibody yields a sample indistinguishable from those that did not receive hPRL agonist. The percentage of maximum hPRLR activity may be plotted against the antibody concentrations to generate a concentration-response curve, which may be subsequently used to determine a concentration-based value (IC₅₀) used to further quantify the ability of an antibody or antigen-binding fragment thereof to neutralize hPRL agonism.

In another aspect of the invention, there is provided a composition comprising an antibody or antigen-binding fragment described herein, for use as a medicament.

In another aspect of the invention, there is provided the use of a composition described herein for use in the manufacture of a medicament, for example a medicament for the treatment of a disease or condition which is modulated by hPRL. Optionally, the medicament may be for the treatment of acute pain, neuropathic peripheral pain, chronic pain, and/or osteoarthritis.

In another aspect of the invention, there is provided a method of treatment of a disease or condition which is modulated by hPRL, comprising a step of administering to a patient an effective amount of an antibody or antigen-binding fragment thereof described herein. Optionally, the method of treatment may be a method of treatment of acute pain, neuropathic peripheral pain, chronic pain, or osteoarthritis.

In another aspect of the invention, there is provided an antibody or antigen-binding fragment described herein or pharmaceutical composition thereof for use in therapy. In some embodiments, the present invention provides an antibody of the present invention or pharmaceutical composition thereof for use in the treatment of pain.

In another aspect of the invention, there is provided nucleic acid molecules encoding the antibody of the present invention. In an embodiment, the present invention provides a DNA molecule comprising a polynucleotide sequence encoding a HC, wherein the amino acid sequence of the HC is SEQ ID NO: 1. According to some such embodiments, the DNA molecule has a polynucleotide sequence given by SEQ ID NO: 12.

In another aspect of the invention, there is provided a DNA molecule comprising a polynucleotide sequence encoding a LC, wherein the amino acid sequence of the LC is SEQ ID NO: 2. According to some such embodiments, the DNA molecule has a polynucleotide sequence given by SEQ ID NO: 13.

In another aspect of the invention, there is provided a DNA molecule comprising a polynucleotide sequence encoding a HC having the amino acid sequence of SEQ NO: 1, and comprising a polynucleotide sequence encoding a LC having the amino acid sequence of SEQ ID NO: 2. In a particular embodiment, the polynucleotide sequence encoding the HC having the amino acid sequence of SEQ ID NO: 1 is given by SEQ ID NO: 12, and the polynucleotide sequence encoding the LC having the amino acid sequence of SEQ ID NO: 2 is given by SEQ ID NO: 13.

In another aspect of the invention, there is provided a mammalian cell transformed with DNA molecule(s), in which the cell is capable of expressing a compound comprising a HC and a LC of the present invention, wherein the HC is given by SEQ ID NO: 1, and the LC is given by SEQ ID NO: 2.

Also, the present invention provides a process for producing a compound comprising the HC and the LC, comprising cultivating the mammalian cell under conditions such that the antibody of the present invention is expressed. The present invention also provides an antibody produced by said process.

In another aspect of the invention, there is provided an antibody or antigen-binding fragment that contacts hPRL at a epitope, wherein the epitope includes the following residues of SEQ ID NO: 11: Thr at position 7; Arg at position 10; Tyr at position 58; Arg at position 65; Ser at position 76; Thr at position 85; Ser at position 117; and Ser at position 125. In such an embodiment, the epitope is determined by cross-linking high-mass MALDI mass spectrometry and nLC-Q-Exactive Plus tandem mass spectrometry.

In a particularly preferred aspect of the invention, the antibody is Antibody 37 or antigen-binding fragment thereof. Herein, "Antibody 37" is understood to mean the antibody expressed and purified as per *Example 1* below. In the following, characteristics of "Antibody 37" are described by way of example: Antibody 37 binds to hPRL. Antibody 37 comprises a LC comprising LCDR1, LCDR2, and LCDR3. LCDR1 has an amino acid sequence selected from SEQ ID NO: 8. LCDR2 has an amino acid sequence selected from SEQ ID NO: 9. LCDR3 has an amino acid sequence selected from SEQ ID NO: 10. Antibody 37 comprises a HC comprising HCDR1, HCDR2, and HCDR3. HCDR1 has an amino acid sequence selected from SEQ ID NO: 5. HCDR2 has an amino acid sequence selected from SEQ ID NO: 6. HCDR3 has an amino acid sequence selected from SEQ ID NO: 7. Antibody 37 comprises a LCVR with an amino acid sequence selected from SEQ ID NO: 4. Antibody 37 comprises a HCVR with the amino acid sequence selected from SEQ ID NO: 3. Antibody 37 binds an epitope having an amino acid sequence selected from SEQ ID NO: 11. Antibody 37 is an antibody of IgG4(S228P).

### List of Figures

Embodiments of the invention will now be described, by way of illustration only, with reference to the accompanying figures, in which:
**Figure 1** is a line graph showing the binding and dissociation kinetics for Antibody 37 binding varying concentrations of hPRL.
**Figure 2** is a graph showing the hPRLR activity assay with Antibody 37.

### Detailed description

### Methods

The antibody or antigen-binding fragment of the present invention may be prepared and purified using known methods. For example, cDNA sequences encoding the HC (amino acid sequence given by SEQ ID NO: 1) and LC (amino acid sequence given by SEQ ID NO: 2) may be cloned and engineered into a glutamine synthetase (GS) expression vector. The engineered immunoglobulin expression vector may then be stably transfected into CHO cells. As one skilled in the art will appreciate, mammalian expression of antibodies will result in glycosylation, typically at highly conserved N-glycosylation sites in the Fc region. Stable clones may be verified for expression of an antibody specifically binding to hPRL. Positive clones may be expanded into chemically defined serum-free culture medium for antibody production in bioreactors. An antibody secreted into medium may be purified by conventional techniques. For example, the medium may be conveniently applied to a Protein A or G Sepharose FF column that has been equilibrated with a compatible buffer, such as sodium phosphate (pH 7.0). The column is washed to remove nonspecific binding components. The bound antibody is eluted, for example, by pH gradient and antibody fractions are detected such as by reverse phase chromatography, and then pooled. The antibody may be concentrated and/or sterile filtered using common techniques. Soluble aggregate and multimers may be effectively removed by common techniques, including size exclusion, hydrophobic interaction, ion exchange, or hydroxyapatite chromatography. The product may be immediately frozen, for example at -80°C, or may be lyophilized.

The antibody or antigen-binding fragment may be incorporated into a pharmaceutical composition which can be prepared by methods well known in the art and comprise an antibody of the present invention and one or more pharmaceutically acceptable carriers, diluents, or excipients.

### Antibody Engineering

A parental murine monoclonal anti-hPRL antibody was generated by mouse immunization with hPRL followed by hybridoma generation and screening using well-characterized methods. The CDRs of the parental antibody were sequenced and a humanized anti-hPRL antibody was optimized using a framework library approach. For the framework library, four human VH framework germlines genes (IGHV1-46, IGHV1-69, IGHV1-3, IGHV1-46*01/4m) and four human VL framework genes (IGKV7-3, IGKV4-1, IGKV1-39, IGKV7-3*01/4m) containing anti-hPRL CDRs were synthesized and cloned into HC and LC human IgG1 expression vectors. Following transient transfection of all 16 HC and LC combinations into HEK 293 cells, supernatants were assayed by ELISA for binding to hPRL directly coated onto a plate. Purified antibodies were also screened for neutralization of hPRL activation of hPRLR by *in vitro* activity assay. Humanized antibodies with CDRs derived from the parental murine antibody, utilizing the IGHV1-3 or IGHV1-46 HC human framework and IGKV1-39 or IGKV4-1 human LC framework, were chosen for further development. cDNA sequences for the selected variable regions with IgG1 or IgG4 (with well-known hinge stability mutation) HC constant regions were cloned into GS expression vectors. Selected antibody candidates were compared by expression titers, purification yields, neutralization activity by *in vitro* activity assay, and affinity to human, cynomolgus monkey, and mouse PRL. Overall, Antibody 37, a humanized anti-hPRL antibody utilizing IGHV1-3 human HC framework and IGKV1-39 human LC framework with human IgG4 HC constant region with well-known hinge mutation had preferred properties as compared to the other candidates.

### Experimental Examples

### Example 1: Antibody Expression and Purification

The antibody of the invention can be biosynthesized, purified, and formulated for administration by well-known methods. Appropriate hosts cells are commercially available at Lonza Bioscience. For example, an appropriate host cell, such as HEK 293 or CHO, is either transiently or stably transfected with an expression system for secreting antibodies using a predetermined HC:LC vector ratio if two vectors are used, or a single vector system encoding both HC and LC. Vectors suitable for expression and secretion of antibodies from these commonly used host cells are well-known.

Following expression and secretion of the antibody, the medium is clarified to remove cells and purified using any of many commonly used techniques. For example. The medium may be applied to a Protein A or G column that has been equilibrated with buffer, such as sodium phosphate (pH 7.0). The column is washed to remove nonspecific binding components. The bound antibody is eluted, for example, by a pH gradient (such as 0.1 M sodium phosphate buffer 6.8 to 0.1 M sodium citrate buffer pH 2.5). Antibody fractions are detected, such as by reverse phase chromatography, and then are pooled. Further purification is optional, depending on the intended use. The antibody may be concentrated and/or sterile filtered using common techniques. Other materials than the antibody, such as host cell and growth medium components, and soluble aggregates and multimers of the antibody, may be effectively reduced or removed by common techniques, including size exclusion, hydrophobic interaction, ion exchange, or hydroxyapatite chromatography. The product may be immediately frozen, for example at -80°C, or may be lyophilized.

Exemplified Antibody 37 was expressed either transiently in HEK 293 cells after co-transfection of separate HC and LC expression DNA vectors that incorporate the DNA sequences of SEQ ID NO: 12 and SEQ ID NO: 13 respectively, or was expressed stably in CHO cells after transfection of a single DNA vector that incorporated the DNA sequence of both SEQ ID NO: 12 and SEQ ID NO: 13, which encode the HC and LC, respectively. Medium harvested from either a 6-day transient HEK 293 culture or a 15-day CHO fed-batch culture was clarified, and the resulting crude supernatant purified by Protein A chromatography. Antibody 37 bound to Protein A resin was eluted using low pH buffer. The eluted antibody was further purified by size exclusion chromatography. The final purity of Antibody 37 was evaluated using analytical size exclusion chromatography and LC/MS analysis. Purified Antibody 37 was stored in phosphate buffered saline, pH 7.4 at 4°C.

### Example 2: In vitro Binding Affinity and Kinetics.

The binding kinetics and affinity of Antibody 37 to human, cynomolgus monkey, and mouse PRL was determined using bio-layer interferometry on a ForteBio Octet-RED96 instrument. Anti-human IgG Fc Capture sensors were used as a capture system. Antibody 37 was diluted to 5 µg/mL. Human, cynomolgus monkey, and mouse PRL were prepared at final concentrations of 100, 33, and 11 nM. Each analysis cycle involves the steps of (1) capturing antibody onto the sensor, (2) quenching open binding sites with 150 µg/mL control human gamma globulin, (3) lowering the sensor into PRL for 600s, and (4) returning to buffer for 600s to monitor antigen dissociation, (5) regenerating the sensor surface and repeating the cycle. Biomolecular interaction analysis is performed in ForteBio Data Analysis software using a 1:1 binding model to determine the association rate (on-rate, kₒₙ, M⁻¹s⁻¹ units) and the dissociation rate (off-rate, k_{off}, s⁻¹ units). The equilibrium dissociation constant (K_{D}) is calculated from the relationship K_{D}=k_{off}/kₒₙ and is in molar units.

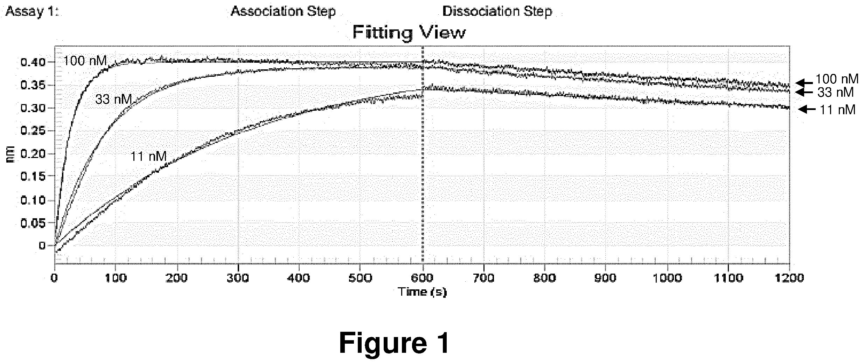

**Figure 1** shows the binding and dissociation kinetics of Antibody 37 for hPRL. More specifically, **Figure 1** shows the curves representing Antibody 37 binding to and dissociating from 11, 33, and 100 nM hPRL are shown, together with best fit curves. Antibody 37 shows rapid binding to hPRL, which is dependent upon the antigen concentration. The best fit curve for binding was used to determine the rate of association (kₒₙ, expressed in M⁻¹s⁻¹ units). Following binding, the sensor with immobilized Antibody 37 and bound hPRL is moved into buffer to monitor the rate of dissociation. Antibody 37 shows a very slow dissociation over time, indicative of strong binding to hPRL (k_{off}, expressed in s⁻¹). Binding curves for cynomolgus monkey PRL and mouse PRL are not shown. Antibody 37 shows similar binding kinetics and affinity for cynomolgus monkey PRL and hPRL. Interaction between Antibody 37 and mouse PRL showed limited response above background, and thus kinetic measurements were not determined (ND). It is therefore assumed that the K_{D} value for mouse PRL concentration higher than the maximum concentration tested (100 nM). Together, these results show that Antibody 37 has high affinity for both hPRL and cynomolgus PRL, while exhibiting limited cross-reactivity with mouse PRL.

**Table 2** below lists the PRL binding kinetics and affinity of antibody of the present invention determined from a global fit of the curves represented in **Figure 1** for hPRL.

**Table 2**

| **Antigen** | **kₒₙ (M⁻¹s⁻¹)** | **k_{off} (s⁻¹)** | **K_{D} (pM)** | **N** |
|---|---|---|---|---|
| **Human PRL** | 3.47 x 10⁵ | 2.15 x 10⁻⁴ | 621 | 1 |
| **Cynomolgus PRL** | 5.43 x 10⁵ | 3.07 x 10⁻⁴ | 565 | 1 |
| **Mouse PRL** | ND | ND | >100,000 | 1 |

### Example 3: Neutralization of hPRL activity in the CHO-hPRLR phospho-STATS cell signaling assay in vitro

CHO cells stably expressing PRLR were used to determine the ability of Antibody 37 to inhibit PRL-induced activity. In response to PRL, PRLR activates the JAK2/STAT5 pathway. This activity can be measured using a fluorescence resonance energy transfer (FRET) assay designed to detect phosphorylated STAT5.

Cells were treated with hPRL, mouse PRL, cynomolgus monkey PRL, human growth hormone (GH), or human placental lactogen (PL) in the presence or absence of Antibody 37. For each test, Antibody 37 was pre-incubated with PRL or other hormone for 30 minutes at room temperature. Prior to treatment, CHO-PRLR cells were plated at 100,000 cells in 8 µL per well in white, low-volume 384 well assay plates. The plated cells were then treated with 4 µl of the antibody-PRL mixture is added to each well in triplicate. hPRL was added at a consistent 20 nM dose and the dose range of Antibody 37 was 0-150 nM. Culture medium alone was used as a no treatment control. The assay plates were then sealed with a porous plate sealer and incubated for 15 minutes at 37°C with 95% relative humidity and 5% CO₂. Following incubation, cells were lysed by adding 3 µl of lysis buffer (Bioauxilium) supplemented with phosphatase inhibitor cocktail to each well, resealed with a plate sealer, and incubated for 30 minutes at room temperature with gentle shaking (400 rpm). After lysis, 5 µL diluted detection antibody mixtures (Bioauxilium) were added to each well. Assay plates were sealed and incubated at RT in the dark overnight. After incubation, plate sealers were removed from assay plates and fluorescence intensity TR-FRET signal was measured at 620 nm and 665 nm excitation using a TECAN SPARK multimode plate reader. The results are shown below in Table 3 (Antibody 37 IC₅₀ in the *in vitro* CHO-hPRLR phospho-STAT5 cell signaling assay).

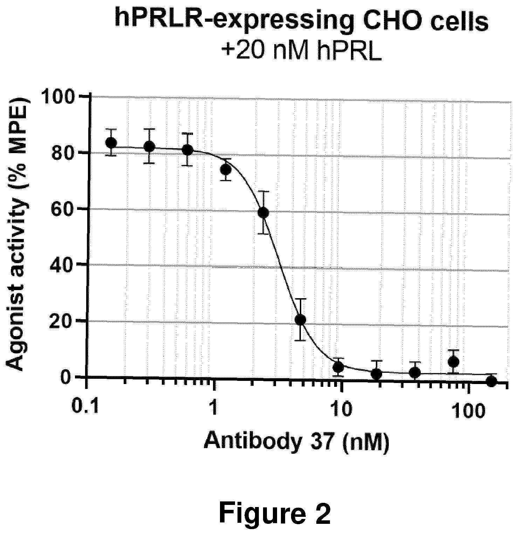

The results of the hPRLR activity assay are shown in **Figure 2****,** which shows the hPRLR activation by 20 nM hPRL upon addition of various concentrations of Antibody 37. IC₅₀ determinations of Antibody 37 neutralization of hPRL and other agonists are presented in **Table 3.**

**Table 3**

| **Type** | **IC₅₀(nM)** |
|---|---|
| Human PRL | 3.1 |
| Cynomolgus PRL | 4.7 |
| Mouse PRL | >150 |
| Human GH | >150 |
| Human PL | >3,622 |

### Example 4: Epitope mapping by cross-linking mass spectrometry

The epitope recognized on hPRL antigen by Antibody 37 was determined using the high-resolution method developed by CovalX. A 5 µM/1.25 µM mixture of hPRL/Antibody 37 was incubated with deuterated crosslinker before undergoing multi-enzymatic cleavage. 1 µL of control and crosslinked solutions were loaded onto an UltiMate 3000-RSLC nano-liquid chromatography system in line with a Q-Exactive Plus Orbitrap mass spectrometer. Spectra generated were analyzed using XQuest and Stavrox software. Crosslinking analysis showed that the epitope on hPRL recognized by Antibody 37 was discontinuous and comprised the following amino acid residues of SEQ ID NO: 11: T7, R10, Y58, R65, S76, T85, S117, and S125.

### Overview of Sequences

Reference is made to the following sequences, in which:
HC relates to **SEQ ID NO: 1;**
LC relates to **SEQ ID NO: 2;**
HCVR relates to **SEQ ID NO: 3;**
LCVR relates to **SEQ ID NO: 4;**
HCDR1 relates to **SEQ ID NO: 5;**
HCDR2 relates to **SEQ ID NO: 6;**
HCDR3 relates to **SEQ ID NO: 7;**
LCDR1 relates to **SEQ ID NO: 8;**
LCDR2 relates to **SEQ ID NO: 9;**
LCDR3 relates to **SEQ ID NO: 10;**
Human PRL Amino Acids 39 - 199 relates to **SEQ ID NO: 11;**
DNA Encoding the HC of **SEQ ID NO:1** relates to **SEQ ID NO:12;**
DNA Encoding the LC of **SEQ ID NO: 2** relates to **SEQ ID NO:13.**

More specifically, reference is made to the following sequences, in which: HC of Antibody 37 is **SEQ ID NO: 1,** LC of Antibody 37 is **SEQ ID NO: 2,** HCVR of Antibody 37 is **SEQ ID NO: 3,** LCVR of Antibody 37 is **SEQ ID NO: 4,** HCDR1 is **SEQ ID NO: 5,** HCDR2 is **SEQ ID NO: 6,** HCDR3 is **SEQ ID NO: 7,** LCDR1 is **SEQ ID NO: 8,** LCDR2 is **SEQ ID NO: 9,** LCDR3 is **SEQ ID NO: 10,** Human PRL Amino Acids 39 - 199 is **SEQ ID NO: 11,** DNA Encoding the HC of **SEQ ID NO:1** is **SEQ ID NO:12,** and/or DNA Encoding the LC of **SEQ ID NO: 2** is **SEQ ID NO:13.**

The following sequence listing has also been filed in the applicable WIPO Standard ST.26.

### Sequence listing

**SEQ ID NO: 1** 
QVQLVQSGAEVKKPGASVKVSCKASGYTFSNFWIEWVRQAPGQRLEWMGEIFPGTGSTYYTEKFK
VRVTITRDTSASTAYMELSSLRSEDTAVYYCARRGYSDSWFAHWGQGTLVTVSSASTKGPSVFPLA
PCSRSTSESTAALGCLVKDYFPEPVTVSWNSGALTSGVHTFPAVLQSSGLYSLSSVVTVPSSSLGT
KTYTCNVDHKPSNTKVDKRVESKYGPPCPPCPAPEFLGGPSVFLFPPKPKDTLMISRTPEVTCVVV
DVSQEDPEVQFNWYVDGVEVHNAKTKPREEQFNSTYRVVSVLTVLHQDWLNGKEYKCKVSNKGL
PSSIEKTISKAKGQPREPQVYTLPPSQEEMTKNQVSLTCLVKGFYPSDIAVEWESNGQPENNYKTTP
PVLDSDGSFFLYSRLTVDKSRWQEGNVFSCSVMHEALHNHYTQKSLSLSLGK
**SEQ ID NO: 2** 
DIQMTQSPSSLSASVGDRVTITCRASESVDMYGKSFMHWYQQKPGKAPKLLIYRASTLESGVPSRF
SGSGSGTDFTLTISSLQPEDFATYYCQQNVEAPLTFGGGTKVEIKRTVAAPSVFIFPPSDEQLKSGTA
SVVCLLNNFYPREAKVQWKVDNALQSGNSQESVTEQDSKDSTYSLSSTLTLSKADYEKHKVYACEV
THQGLSSPVTKSFNRGEC
**SEQ ID NO: 3** 
QVQLVQSGAEVKKPGASVKVSCKASGYTFSNFWIEWVRQAPGQRLEWMGEIFPGTGSTYYTEKFK
VRVTITRDTSASTAYMELSSLRSEDTAVYYCARRGYSDSWFAHWGQGTLVTVSS
**SEQ ID NO: 4** 
DIQMTQSPSSLSASVGDRVTITCRASESVDMYGKSFMHWYQQKPGKAPKLLIYRASTLESGVPSRF
SGSGSGTDFTLTISSLQPEDFATYYCQQNVEAPLTFGGGTKVEIK
**SEQ ID NO**: **5**
   YTFSNFWIE
**SEQ ID NO: 6**
   EIFPGTGSTYYTEKFKV
**SEQ ID NO: 7**
   RGYSDSWFAH
**SEQ ID NO: 8**
   RASESVDMYGKSFMH
**SEQ ID NO: 9**
   RASTLES
**SEQ ID NO: 10**
   QQNVEAPLT
**SEQ ID NO: 11** 
EFDKRYTHGRGFITKAINSCHTSSLATPEDKEQAQQMNQKDFLSLIVSILRSWNEPLYHLVTEVRGM
QEAPEAILSKAVEIEEQTKRLLEGMELIVSQVHPETKENEIYPVWSGLPSLQMADEESRLSAYYNLLH
CLRRDSHKIDNYLKLLKCRIIHNNNC
**SEQ ID NO:12** 
CAAGTGCAGCTGGTGCAGAGCGGCGCCGAGGTGAAGAAGCCTGGCGCTAGCGTGAAGGTGA
GCTGCAAGGCTAGCGGCTACACCTTCAGCAACTTCTGGATCGAGTGGGTGAGACAAGCCCCTG
GACAGAGACTGGAGTGGATGGGCGAGATCTTCCCTGGCACCGGCAGCACCTACTACACCGAG
AAGTTCAAGGTGAGAGTGACCATCACAAGAGACACAAGCGCGTCCACCGCCTACATGGAGCTG
AGCAGCCTGAGAAGCGAGGACACCGCCGTGTACTACTGCGCTAGAAGAGGGTATTCAGATAGC
TGGTTCGCCCACTGGGGGCAAGGCACTCTGGTGACCGTGAGCAGCGCTAGCACCAAAGGTCC
TAGCGTGTTCCCTCTAGCCCCTTGCAGCAGAAGCACAAGCGAGAGCACCGCCGCCCTGGGCT
GCCTGGTGAAAGACTATTTCCCTGAGCCTGTGACCGTGAGTTGGAACAGCGGCGCCCTGACAA
GCGGCGTGCACACCTTCCCTGCCGTGCTGCAGAGCAGCGGCCTGTACAGCCTGAGCAGCGTG
GTGACCGTGCCAAGCAGCAGCCTGGGCACCAAGACCTACACCTGCAACGTGGACCACAAGCC
TAGCAACACCAAGGTGGACAAGAGAGTGGAGAGCAAGTACGGTCCTCCGTGTCCCCCGTGCC
CTGCCCCTGAGTTCCTGGGCGGCCCTAGCGTCTTTCTGTTCCCACCTAAGCCTAAGGACACCC
TGATGATCAGCAGAACCCCTGAGGTGACCTGCGTGGTGGTGGACGTGAGCCAAGAGGACCCT
GAGGTGCAGTTCAACTGGTACGTGGACGGCGTGGAGGTGCACAACGCCAAGACCAAGCCTAG
AGAGGAGCAGTTCAACAGCACCTACAGAGTGGTGAGCGTGCTGACCGTGCTGCACCAAGACTG
GCTGAACGGCAAGGAGTACAAGTGCAAGGTGAGCAACAAGGGCCTGCCTAGTTCCATTGAGAA
GACCATCAGCAAGGCCAAGGGACAGCCTAGAGAGCCTCAAGTGTACACCCTGCCTCCTAGCCA
AGAGGAGATGACCAAGAACCAAGTGAGCCTGACCTGCCTGGTAAAGGGGTTCTACCCTAGCGA
CATCGCCGTGGAGTGGGAGAGCAACGGACAGCCTGAGAACAACTACAAGACCACCCCTCCTGT
GCTGGACAGCGACGGCAGCTTCTTCCTGTACAGCAGACTGACCGTGGACAAGAGCAGATGGC
AAGAGGGCAACGTGTTCAGCTGCAGCGTGATGCACGAGGCCCTGCACAACCACTACACACAGA
AGAGCCTGAGCCTGAGCCTGGGCAAG
**SEQ ID NO:13**

GACATTCAGATGACACAGAGCCCTAGCAGCCTGAGCGCTAGCGTGGGCGACAGAGTGACCAT
CACCTGCAGAGCTAGCGAGAGCGTGGACATGTACGGCAAGAGCTTCATGCACTGGTATCAGCA
GAAGCCTGGCAAGGCCCCTAAGCTGCTGATCTACAGAGCTAGCACCCTGGAGAGCGGCGTGC
CTAGCAGATTCAGCGGCAGCGGCAGCGGCACCGACTTCACCCTGACCATCAGCAGCCTGCAG
CCTGAGGACTTCGCCACCTACTACTGTCAGCAGAACGTGGAGGCCCCTCTGACCTTCGGCGGC
GGCACCAAGGTGGAGATCAAGAGAACCGTGGCCGCCCCTAGCGTGTTCATCTTCCCTCCTAGC
GACGAGCAGCTGAAGAGCGGCACCGCTAGCGTGGTGTGCCTGCTGAACAACTTCTACCCTAGA
GAGGCCAAGGTGCAGTGGAAGGTGGACAACGCCCTGCAGAGCGGCAACAGCCAAGAGAGCGT
GACCGAGCAAGACAGCAAGGACAGCACCTACAGCCTGAGCAGCACCCTGACCCTGAGCAAGG
CCGACTACGAGAAGCACAAGGTGTACGCCTGCGAGGTGACCCACCAAGGCCTGAGCAGCCCT
GTGACCAAGAGCTTCAACAGAGGCGAGTGC

## Claims

1. An antibody or antigen-binding fragment thereof, wherein the antibody or antigen-binding fragment thereof binds to human prolactin (hPRL).

2. The antibody or antigen-binding fragment thereof according to claim 1 comprising a light chain (LC) comprising one or more of LC complementary determining regions (CDRs) LCDR1, LCDR2, and/or LCDR3, wherein
LCDR1 has an amino acid sequence selected from SEQ ID NO: 8,
LCDR2 has an amino acid sequence selected from SEQ ID NO: 9, and/or
LCDR3 has an amino acid sequence selected from SEQ ID NO: 10.

3. The antibody or antigen-binding fragment thereof according to claim 1 or 2 comprising a heavy chain (HC) comprising one or more of HC CDRs HCDR1, HCDR2, and HCDR3, wherein
HCDR1 has an amino acid sequence selected from SEQ ID NO: 5,
HCDR2 has an amino acid sequence selected from SEQ ID NO: 6, and/or
HCDR3 has an amino acid sequence selected from SEQ ID NO: 7.

4. The antibody or antigen-binding fragment thereof according to any preceding claim comprising a LC variable region (LCVR), wherein the LCVR has an amino acid sequence selected from SEQ ID NO: 4, and/or comprising a HC variable region (HCVR), wherein the HCVR has an amino acid sequence selected from SEQ ID NO: 3.

5. The antibody or antigen-binding fragment thereof according to any preceding claim, wherein the antibody or antigen-binding fragment thereof binds an epitope having an amino acid sequence selected from SEQ ID NO: 11.

6. The antibody or antigen-binding fragment thereof according to any preceding claim, wherein the antibody or antigen-binding fragment thereof is an antibody or antigen-binding fragment thereof of Immunoglobulin G (IgG), for example an IgG of subclass IgG1, IgG2, IgG3, and/or IgG4.

7. The antibody or antigen-binding fragment thereof according to any preceding claim, wherein the antibody or antigen-binding fragment thereof binds to PRL with an affinity of approximately 0.6 nM.

8. The antibody or antigen-binding fragment thereof according to any preceding claim, wherein the antibody or antigen-binding fragment thereof neutralizes hPRL-induced activation of hPRLR, for example, at an *in vitro* efficacy of approximately IC₅₀ of 3.1 nM.

9. The antibody or antigen-binding fragment thereof according to any one of claims 1, 4 to 8, wherein
(i) LCDR1 has an amino acid sequence selected from SEQ ID NO: 8 or an amino acid sequence having one, two, three, four or five amino acid substitutions, deletions or additions as compared to SEQ ID NO: 8,
(ii) LCDR2 has an amino acid sequence selected from SEQ ID NO: 9 or an amino acid sequence having one, two, three, four or five amino acid substitutions, deletions or additions as compared to SEQ ID NO: 9,
(iii) LCDR3 has an amino acid sequence selected from SEQ ID NO: 10 or an amino acid sequence having one, two, three, four or five amino acid substitutions, deletions or additions as compared to SEQ ID NO: 10,
(iv) HCDR1 has an amino acid sequence selected from SEQ ID NO: 5 or an amino acid sequence having one, two, three, four or five amino acid substitutions, deletions or additions as compared to SEQ ID NO: 5,
(v) HCDR2 has an amino acid sequence selected from SEQ ID NO: 6 or an amino acid sequence having one, two, three, four or five amino acid substitutions, deletions or additions as compared to SEQ ID NO: 6,
and/or
(vi) HCDR3 has an amino acid sequence selected from SEQ ID NO: 7 or an amino acid sequence having one, two, three, four or five amino acid substitutions, deletions or additions as compared to SEQ ID NO: 7.

10. The antibody or antigen-binding fragment thereof according to any one of claims 1, 4 to 8, wherein
(i) LCDR1 comprises amino acid sequence SEQ ID NO: 8 or an amino acid sequence that is at least 60% identical to SEQ ID NO: 8.
(ii) LCDR2 comprises amino acid sequence SEQ ID NO: 9 or an amino acid sequence that is at least 60% identical to SEQ ID NO: 9,
(iii) LCDR3 comprises amino acid sequence SEQ ID NO: 10 or an amino acid sequence that is at least 60% identical to SEQ ID NO: 10,
(iv) HCDR1 comprises amino acid sequence SEQ ID NO: 5 or an amino acid sequence that is at least 60% identical to SEQ ID NO: 5,
(v) HCDR2 comprises amino acid sequence SEQ ID NO: 6 or an amino acid sequence that is at least 60% identical to SEQ ID NO: 6,
and/or
(vi) HCDR3 comprises amino acid sequence SEQ ID NO: 7 or an amino acid sequence that is at least 60% identical to SEQ ID NO: 7.

11. A pharmaceutical composition comprising the antibody or antigen-binding fragment thereof according to any proceeding claim.

12. The pharmaceutical composition according to claim 11, comprising a pharmaceutically acceptable carrier, excipient, and/or diluent.

13. A composition comprising the antibody or antigen-binding fragment thereof according to any one of claims 1 to 10 for use in the treatment of a disease or condition which is modulated by hPRL, for example, for use in the treatment of pain, e.g., acute pain, neuropathic peripheral pain, chronic pain, and/or osteoarthritis.

14. Use of the composition according to claim 11 or 12 for the manufacture of a medicament.

15. A method of treatment of a disease or condition which is modulated by PRL, for example, a method of treatment of pain, e.g., of acute pain, neuropathic peripheral pain, chronic pain, or osteoarthritis, comprising a step of administering to a patient an effective amount of an antibody or antigen-binding fragment thereof according to any one of preceding claims 1 to 10.
